(19) **Europäisches Patentamt**

**European Patent Office**

**Office européen des brevets**

(11) **EP 1 040 217 B1**

(12) **EUROPEAN PATENT SPECIFICATION**

(45) Date of publication and mention
of the grant of the patent:
**19.03.2003   Bulletin 2003/12**

(51) Int Cl.⁷: $D04H\ 1/54$, $A61F\ 13/15$

(86) International application number:
**PCT/US97/23459**

(21) Application number: **97951755.4**

(22) Date of filing: **22.12.1997**

(87) International publication number:
**WO 99/032698 (01.07.1999 Gazette 1999/26)**

(54) **BREATHABLE WEB MATERIALS HAVING CLOTH-LIKE TEXTURE**

WASSERDAMPFDURCHLÄSSIGE BAHNMATERIALIEN MIT TEXTILÄHNLICHER TEXTUR

MATERIAUX IMPER-RESPIRANTS EN FEUILLE, PRESENTANT UNE TEXTURE DU TYPE
ETOFFE

(84) Designated Contracting States:
**AT BE CH DE DK ES FI FR GB GR IE IT LI LU NL
PT SE**

(43) Date of publication of application:
**04.10.2000   Bulletin 2000/40**

(73) Proprietor: **THE PROCTER & GAMBLE COMPANY
Cincinnati, Ohio 45202 (US)**

(72) Inventors:
 • **FLEMING, James, Michael
   Sheboygan, WI 53083 (US)**
 • **MONSTER, Robert, Willem
   Kobe 658 (JP)**
 • **GRANDISON, Kevin, Eugene,
   Regency Plaza Apt. PH4
   Urb. Baruta, Caracas 1080 (VE)**
 • **KALNITZ, Howard, Jay
   Howards Grove, WI 53083 (US)**
 • **DOBRIN, George, Christopher
   Cincinnati, OH 45040 (US)**
 • **ANDERSON, Barry, Jay
   Cincinnati, OH 45236 (US)**
 • **GOENS, Thomas, Masazi
   Cincinnati, OH 45211 (US)**
 • **PHILLIPS, Donna, Sue
   Aurora, IN 47001 (US)**

(74) Representative: **Hirsch, Uwe Thomas et al
Procter & Gamble European Service GmbH,
Sulzbacher Strasse 40-50
65824 Schwalbach am Taunus (DE)**

(56) References cited:
**WO-A-97/45086**

# EP 1 040 217 B1

**Description**

**FIELD**

The present invention relates to breathable web materials which are soft, cloth-like in texture, and which are cost-effective to produce.

**BACKGROUND**

Absorbent articles such as sanitary napkins, pantiliners, disposable diapers, incontinent briefs, and bandages are designed to absorb and retain liquid and other discharges from the human body and to prevent body and clothing soiling. Web materials provided in the form of sheets or films have previously been used in such absorbent articles. For example, web materials such as plastic films which exhibit an "elastic-like" behavior in the direction of applied elongation without the use of added traditional elastic and which also exhibit a soft, cloth-like texture have been used. See U.S. Pat. No. 5,650,214 issued to Anderson et al. on July 22, 1997 and WO 97/45086.

In addition to elastic-like behavior and soft, cloth-like texture, another important and desirable feature for web materials useful in absorbent articles is good breathability. "Breathability" as used herein refers to moisture vapor transmission rate, i.e., the ability of the article to allow water vapor to escape from inside the article to outside the article in the presence of a gradient of relative humidity. Good breathability is important for wearer comfort and skin health, and a lack of breathability typically results in a hot, stuffy, skin-unfriendly product for the wearer.

Conventional films used in absorbent articles have typically been rendered breathable by a process known as hot tentering, in which a formed film material that has an inclusion of a solid ingredient such as calcium carbonate is drawn at a temperature higher than ambient, thereby causing microporous holes to form in the film. However, hot tentering tends to be an expensive process and generally is performed as a separate process apart from the processing of the absorbent article as a whole; on-line hot tentering would be slow, inefficient and commercially unattractive. In addition, as the breathability of a hot tentered film increases, the likelihood of liquid leakage through such a film also typically increases. This occurs when the microporous holes that are formed in the film are made too large to prevent the passage of liquid.

Based on the foregoing, there is a need for a web material, particularly a plastic film, that is provided with enhanced breathability while being cost-effective to produce. None of the existing art provides all of the advantages and benefits of the present invention.

**SUMMARY**

The present invention is directed to a soft web material comprising: a plurality of first regions and a plurality of second regions being comprised of the same material composition, the first regions surrounding the second regions and the second regions comprising a plurality of raised rib-like elements comprising microporous holes, the web material having been mechanically modified to exhibit a MVTR of at least about 100 $g/m^2/24$ hrs and a MWTR of not greater than about 100 g/hr.

These and other features, aspects and advantages of the invention will become evident to those skilled in the art from a reading of the present disclosure.

**BRIEF DESCRIPTION OF THE DRAWINGS**

While the specification concludes with claims particularly pointing out and distinctly claiming the present invention, it is believed that the present invention will be better understood from the following description of preferred embodiments taken in conjunction with the accompanying drawings, in which like reference numerals identify like elements and wherein:

Fig. 1 is a simplified plan view illustration of a prior art sanitary napkin with portions cut-away to more clearly show the construction of the sanitary napkin;
Fig. 2 is a simplified plan view illustration of a prior art disposable diaper with portions cut-away to more clearly show the construction of the disposable diaper;
Fig. 3 is a plan view illustration of a preferred embodiment of a polymeric web material of the present invention;
Fig. 4 is an exemplary graph of the resistive force versus percent elongation behavior of a web material of the present invention, such as shown in Fig. 3, and a base web material, i.e., which does not include first and second regions, of similar material composition;
Fig. 5 is a plan view illustration of a polymeric web material of Fig. 3 in a tensioned condition corresponding to stage I on the force-elongation curve depicted in Fig. 4;

Fig. 6 is an exemplary graph of the elastic hysteresis behavior of the web material of the present invention which is graphically represented by curve 720 in Fig. 4 when the web material is subjected to a hysteresis test at 60% elongation; and

Fig. 7 is a simplified perspective view of a preferred apparatus used to form web materials of the present invention with a portion of the apparatus being tilted to expose the teeth.

## DETAILED DESCRIPTION

As used herein, the term "absorbent article" refers to devices which absorb and contain body exudates, and, more specifically, refers to devices which are placed against or in proximity to the body of the wearer to absorb and contain the various exudates discharged from the body. The term "absorbent article" is intended to include diapers, catamenial pads, sanitary napkins, pantiliners, incontinent briefs, bandages, and the like. The term "disposable" is used herein to describe absorbent articles which are not intended to be laundered or otherwise restored or reused as an absorbent article (i.e., they are intended to be discarded after a single use, and, preferably, to be recycled, composted or otherwise disposed of in an environmentally compatible manner). Because of their single use nature, low cost materials and methods of construction are highly desirable in disposable absorbent articles.

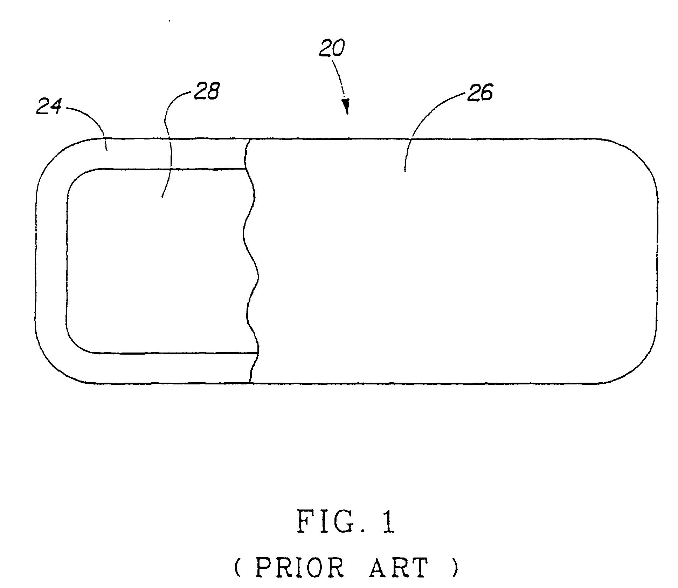

Fig. 1 is a plan view of a prior art sanitary napkin 20 with portions of the structure being cut-away to more clearly show the construction of the sanitary napkin 20 and with the portion of the sanitary napkin 20 which faces away from the wearer, i.e., the outer surface, oriented towards the viewer. As used herein, the term "sanitary napkin" refers to an absorbent article which is worn by females adjacent to the pudendal region, generally external to the urogenital region, and which is intended to absorb and contain menstrual fluids and other vaginal discharges from the wearer's body (e.g., blood, menses, and urine). As shown in Fig. 1, the sanitary napkin 20 comprises a liquid pervious topsheet 24, a liquid impervious backsheet 26 joined with the topsheet 24, and an absorbent core 28 positioned between the topsheet 24 and the backsheet 26.

While the topsheet, backsheet, and absorbent core may be assembled in a variety of well known configurations (including so called "tube" products or side flap products), preferred sanitary napkin configurations are described generally in U.S. Pat. No. 4,950,264, issued to Osborn on Aug. 21, 1990; U.S. Pat. No. 4,425,130, issued to DesMarais on Jan. 10, 1984; U.S. Pat. No. 4,321,924, issued to Ahr on Mar. 30, 1982; and U.S. Pat. No. 4,589,876, issued to Van Tilburg on May 20, 1986.

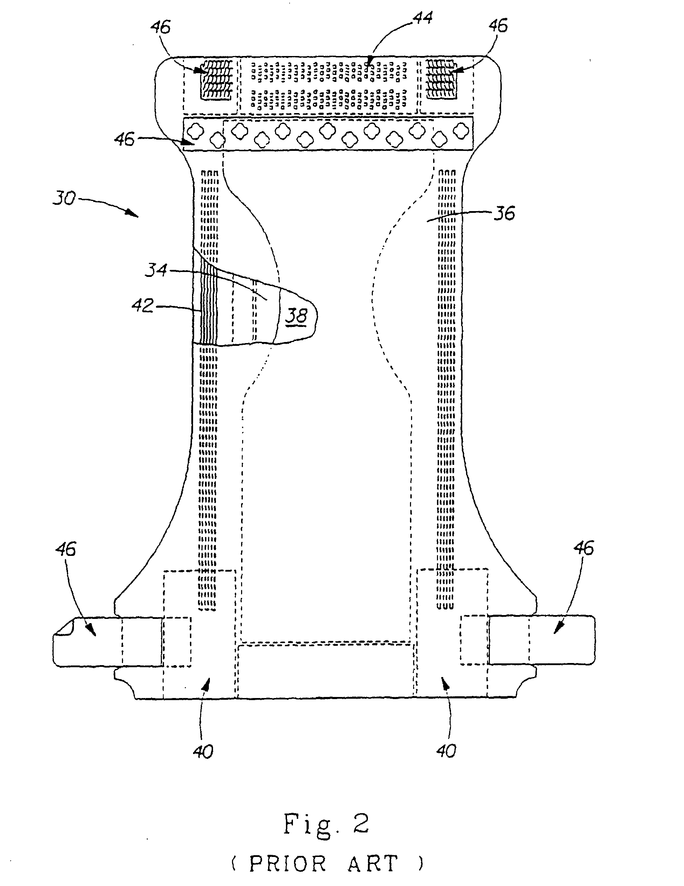

Fig. 2 is a plan view of a prior art disposable diaper 30 in its uncontracted state (i.e., with elastic induced contraction pulled out except in the side panel wherein the elastic is left in its relaxed condition) with portions of the structure being cut-away to more clearly show the construction of the diaper 30 and with the portion of the diaper 30 which faces away from the wearer, i.e., the outer surface, oriented towards the viewer. As used herein, the term "diaper" refers to an absorbent article generally worn by infants and incontinent persons that is worn about the lower torso of the wearer. As shown in Fig. 2, the diaper 30 comprises a liquid pervious topsheet 34, a liquid impervious backsheet 36 joined with the topsheet 34, an absorbent core 38 positioned between the topsheet 34 and the backsheet 36, elasticized side panels 40, elasticized leg cuffs 42, an elastic waist feature 44, and a fastening system generally multiply designated as 46.

While the diaper 30 may be assembled in a variety of well known configurations, preferred diaper configurations are described generally in U.S. Pat. No. 3,860,003, issued to Kenneth B. Buell on Jan. 14, 1975; and U.S. Pat. No. 5,151,092 issued to Kenneth B. Buell et al. on Sept. 29, 1992.

While the present invention will be described in the context of providing a cloth-like, breathable "web material" which is particularly well suited for use as a backsheet, a topsheet and/or an absorbent core or a portion thereof on a disposable absorbent article such as a disposable diaper, sanitary napkin, or bandage the present invention is in no way limited to such application. It may be employed in nearly any application where a relatively low cost breathable web material is desired, e.g., durable articles of apparel, such as exercise clothing, disposable articles of apparel, elastic bandages, upholstery or wrapping material used to cover complex shaped articles, etc. As used herein the term "web material" refers to a sheet-like material, e.g., a topsheet, backsheet, or absorbent core on a disposable absorbent article, a composite or laminate of two or more sheet-like materials and the like. The present invention may be practiced to great advantage in many situations where it is desirable to produce a cloth-like soft, breathable web material. The detailed description of a preferred structure and its use as a backsheet on a sanitary napkin or a disposable diaper will allow one skilled in the art to readily adapt the present invention to other applications.

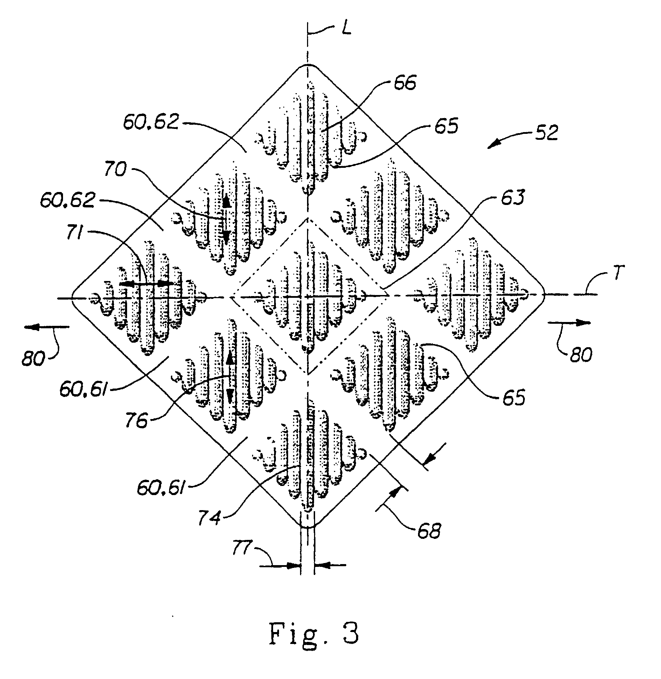

Referring now to Fig. 3, there is shown a preferred embodiment of a polymeric web material 52 of the present invention. The web material 52 is shown in Fig. 3 in its substantially untensioned condition. The web material 52 is particularly well suited for use as a backsheet on an absorbent article, such as the sanitary napkin 20 in Fig. 1 or the disposable diaper 30 in Fig. 2. The web material 52 has two centerlines, a longitudinal centerline, which is also referred to hereinafter as an axis, line, or direction "L" and a transverse or lateral centerline, which is also referred to hereinafter as an axis, line, or direction "T". The transverse centerline "T" is generally perpendicular to the longitudinal centerline "L".

Web material 52 includes a "strainable network" of distinct regions. As used herein, the term "strainable network" refers to an interconnected and interrelated group of regions which are able to be extended to some useful degree in a predetermined direction providing the web material with an elastic-like behavior in response to an applied and subsequently released elongation. The strainable network includes a plurality of first regions 60 and a plurality of second regions 66. Web material 52 also includes transitional regions 65 which are located at the interface between the first regions 60 and the second regions 66. The transitional regions 65 will exhibit complex combinations of the behavior of both the first region and the second region. It is recognized that every embodiment of the present invention will have transitional regions, however, the present invention is largely defined by the behavior of the web material in distinctive regions (e.g., first regions 60 and second regions 66). Therefore, the ensuing description of the present invention will be concerned with the behavior of the web material in the first regions 60 and the second regions 66 only since it is not significantly dependent upon the complex behavior of the web material in the transitional regions 65.

Web material 52 has a first surface, (facing the viewer in Fig. 3), and an opposing second surface (not shown). In the preferred embodiment shown in Fig. 3, the strainable network includes a plurality of first regions 60 and a plurality of second regions 66. A portion of the first regions 60, indicated generally as 61, are substantially linear and extend in a first direction. The remaining first regions 60, indicated generally as 62, are substantially linear and extend in a second direction which is substantially perpendicular to the first direction. While it is preferred that the first direction be perpendicular to the second direction, other angular relationships between the first direction and the second direction may be suitable so long as the first regions 61 and 62 intersect one another. Preferably, the angles between the first and second directions ranges from about 45 degrees to about 135 degrees, with 90 degrees being the most preferred. The intersection of the first regions 61 and 62 forms a boundary, indicated by phantom line 63 in Fig. 3, which completely surrounds the second regions 66.

Preferably, the width 68 of the first regions 60 is from about 0,254 mm to 12,7 mm (0.01 inches to about 0.5 inches) and more preferably from about 0.762 mm to 6,4 mm (0.03 inches to about 0.25 inches). However, other width dimensions for the first regions 60 may be suitable. Because the first regions 61 and 62 are perpendicular to one another and equally spaced apart, the second regions have a square shape. However, other shapes for the second region 66 are suitable and may be achieved by changing the spacing between the first regions and/or the alignment of the first regions 61 and 62 with respect to one another. The second regions 66 have a first axis 70 and a second axis 71. The first axis 70 is substantially parallel to the longitudinal axis of the web material 52, while the second axis 71 is substantially parallel to the transverse axis of the web material 52. The first regions 60 have an elastic modulus E1 and a cross-sectional area A1. The second regions 66 have an elastic modulus E2 and a cross-sectional area A2.

In the illustrated embodiment, the web material 52 has been "formed" such that the web material 52 exhibits a resistive force along an axis, which in the case of the illustrated embodiment is substantially parallel to the transverse axis of the web, when subjected to an applied axial elongation in a direction substantially parallel to the transverse axis. As used herein, the term "formed" refers to the creation of a desired structure or geometry upon a web material that will substantially retain the desired structure or geometry when it is not subjected to any externally applied elongations or forces. A web material of the present invention is comprised of a plurality of first regions and a plurality of second regions, wherein the first regions are visually distinct from the second regions. As used herein, the term "visually distinct" refers to features of the web material which are readily discernible to the normal naked eye when the web material or objects embodying the web material are subjected to normal use. As used herein the term "surface-pathlength" refers to a measurement along the topographic surface of the region in question in a direction substantially parallel to an axis. The method for determining the surface-pathlength of the respective regions can be found in the Test Methods section set forth in subsequent portions of the present specification.

Methods for forming web materials of the present invention include, but are not limited to embossing by mating plates or rolls, thermoforming, high pressure hydraulic forming, or casting. While the entire portion of the web 52 has been subjected to a forming operation, the present invention may also be practiced by subjecting to formation only a portion thereof, e.g., a portion of a diaper backsheet, as will be described in detail below.

In the preferred embodiment shown in Fig. 3 the first regions 60 are substantially planar. That is, the material within the first regions 60 is in substantially the same condition before and after the formation step undergone by web 52. The second regions 66 include a plurality of raised rib-like elements 74. The rib-like elements 74 may be embossed, debossed or a combination thereof. The rib-like elements 74 have a first or major axis 76 which is substantially parallel to the longitudinal axis of the web 52 and a second or minor axis 77 which is substantially parallel to the transverse axis of the web 52.

The rib-like elements 74 in the second region 66 may be separated from one another by unformed areas, essentially unembossed or debossed, or simply formed as spacing areas. Preferably, the rib-like elements 74 are adjacent to one another and are separated by an unformed area of less than 0.10 inches as measured perpendicular to the major axis 76 of the rib-like elements 74, and more preferably, the rib-like elements 74 are contiguous having no unformed areas between them.

The first regions 60 and the second regions 66 each have a "projected pathlength". As used herein the term "projected pathlength" refers to the length of a shadow of a region that would be thrown by parallel light. The projected pathlength of the first region 60 and the projected pathlength of the second region 66 are equal to one another.

The first region 60 has a surface-pathlength, L1, less than the surface-pathlength, L2, of the second region 66 as measured topographically in a parallel direction while the web is in an untensioned condition. Preferably, the surface-pathlength of the second region 66 is at least about 15% greater than that of the first region 60, more preferably at least about 30% greater than that of the first region, and most preferably at least about 70% greater than that of the first region. In general, the greater the surface-pathlength of the second region, the greater will be the elongation of the web before encountering the force wall.

The breathability of the web materials 52 of the present invention is created via a different mechanism than that used for the conventional microporous films. Instead of a hot tentering process, a mechanical modification of the film or of the film/nonwoven composite, i.e., an on-line activation process which textures the film to create breathability, is performed. Although certain previously described web materials (e.g., as described in Anderson et al. U.S. patent 5,650,214) can provide good elastic behavior and soft cloth-like feel, they may not be able to provide a desired level of breathability without leakage or good cost effectiveness. In addition, the off-line hot tentering process that is used to create the breathability of such web materials is typically slower and more expensive that of the process of the present invention.

The process of the present invention provides the breathability of the web material because the mean vapor transmission rate (MVTR) of the web material is increased without a corresponding increase in its mean water transmission rate (MWTR), as explained in more detail below. Increased precision and uniformity in the creation of the microporous holes is also achieved, because the engagment and thus the rupturing of the web occurs on a localized level rather than in relation to the entire width of the film. Finally, improved manufacturing cost effectiveness is attained since the process of the present invention can be performed on-line at high line speed.

Mean vapor transmission rate ("MVTR") is a characteristic measure of breathability and "microclimate" of a web material. MVTR refers to the moisture vapor transfer rate from one side of the web material to the other side of the web material per area unit (e.g., per square meter) and per time unit (e.g., per one day). High MVTR is desirable for good skincare because the air can be well ventilated from one side of the web material to the other, e.g., between the inside and the outside of a diaper or sanitary napkin. However, if the MVTR is too high, the risk of odor, noticeable moisture leakage, or both is present. The method for generating the value of MVTR can be found in the Test Method section set forth in a subsequent portion of this description.

Mean water transmission rate ("MWTR") is another characteristic measure used in describing the properties of a web material. MWTR refers to the water transfer rate from one side of the web material to the other side of the web material per time unit (e.g., per hour or per 24 hours). Low MWTR is desirable for the prevention of leaks. The method for generating the value of MWTR can be found in the Test Method section set forth in a subsequent portion of this description.

It is believed that a preferred upper limit for MWTR that represents the level of urine leakage that is consumer-acceptable is about 100g/hr.

Conventional breathable web materials have typically been rendered breathable by adding a specified amount of calcium carbonate to a polyolefin resin, extruding the mixture and then casting the extruded mixture as a thin film. After casting, the film is drawn at a temperature higher than ambient. This process is known as hot tentering. During hot tentering, microporous holes are created in the film by the calcium carbonate particles. However, the control of the size of the microporous holes formed in the film, and correspondingly the control of the breathability of the film, depends in large part upon the grade and purity of calcium carbonate used as well as upon the control of the tentering conditions. For example, larger calcium carbonate particle sizes will create larger microporous holes, potentially leading to liquid leakage; similarly, nonuniform particle sizes or high levels of contaminants may create nonuniformly sized microporous holes, leading to less precision in the microporous structure and less uniform breathability.

The size (i.e., width) of the parent roll of film that can be rendered breathable is also a process limitation inherent in the conventional hot tentering process. Typically, for a 1 meter wide parent roll, no more than a 1.8 meter width full tentered execution can be made. This is due to the loss of control of the tentering process for greater widths, since during tentering only the free ends of the web are held during the draw time. (For conventional blown or cast films, widths of up to 3 meters may typically be processed.)

However, the process of the present invention is advantageous in that no limitation regarding the processable width of the parent roll is inherent. In the process of the present invention, the mechanical modification of the film occurs as a localized rupturing of the parent film and is performed without the need for tension control of the parent film as a whole. Thus, because there is no need to control tension, there is no limit imposed upon the processable width of the film. Thus, manufacturing is made more efficient with the present invention.

Another drawback associated with the conventional hot tentering process is slower line speed. Hot tentering tends to be a somewhat expensive process and is typically performed as a separate process from the overall processing of the absorbent article. Therefore, to incorporate a hot tentering process in, for example, absorbent article production would be commercially unattractive since it would greatly slow the overall line operating speed.

In operating lines in which conventional hot tentered breathable films are produced, about 111 meter/mm [370 feet per minute (ft/min)] is the maximum speed at which the line may be run. As will be understood by those of skill in the art, it is difficult to produce conventional films at higher line speeds, since degradation of conventional materials generally increases as a function of increased line speeds. Thus, as line speeds for production of hot tentered microporous films increase, the potential for liquid (urine) leakage substantially increases when such films are used as a barrier layer in absorbent articles.

The ratio MV/MW for a given material represents the "trade-off* between breathability and leakage of that material. In other words, as the value of this ratio decreases, for a given value of MVTR, the amount of urine leakage increases, leading to a less desirable product from the consumer standpoint. For conventional processes, it is believed that at line speeds of greater than 111 meter/mm (370 ft/min) a decrease in MV/MW is observed, with a marked decrease at speeds of 750 ft/min and faster. Thus, conventional lines are typically not run at speeds of greater than about 370 ft/min.

With the process of the present invention, line speeds of greater than about 370 ft/min result in no substantial decrease in the value of the MV/MW ratio. Thus, line speed can be increased without a reduction in the consumer benefits of breathability with minimal liquid leakage, making the present invention more cost effective than conventional processes. The web materials of the present invention preferably have values of MV\MW greater than about 0.016.

The web materials of the present invention deliver improved skin health without product leakage as compared to conventional web materials. As MVTR increases, ventilation of the wearer's skin improves, and incidence of redness, irritation, heat rash, and diaper rash decrease.

The process of the present invention is further advantageous in that "zones" of varying breathability may be created as desired. For example, in a typical disposable diaper that has been fully loaded with urine, the area typically lowest in urine loading concentration is the back waist area of the diaper. In the back waist area, a wearer has a high concentration of sweat glands in a relatively flat surface area. Thus, in the back waist region, a high MVTR may be desirable, and a corresponding increase in MWTR may be acceptable in this region, while at the same time not compromising MWTR in the crotch region.

Web material 52 exhibits a modified "Poisson lateral contraction effect" substantially less than that of an otherwise identical base web of similar material composition, i.e., a web having no first and second regions. The method for determining the Poisson lateral contraction effect of a material can be found in the Test Methods section set forth in subsequent portions of the present specification. Preferably, the Poisson lateral contraction effect of webs of the present invention is less than about 0.4 when the web is subjected to about 20% elongation. Preferably, the webs exhibit a Poisson lateral contraction effect less than about 0.4 when the web is subjected to about 40, 50 or even 60% elongation. More preferably, the Poisson lateral contraction effect is less than about 0.3 when the web is subjected to 20, 40, 50 or 60% elongation. The Poisson lateral contraction effect of webs of the present invention is determined by the amount of the web material which is occupied by the first and second regions, respectively. As the area of the web material occupied by the first region increases the Poisson lateral contraction effect also increases. Conversely, as the area of the web material occupied by the second region increases the Poisson lateral contraction effect decreases. Preferably, the percent area of the web material occupied by the first region is from about 2% to about 90%, and more preferably from about 5% to about 50%.

Web materials of the prior art which have at least one layer of an elastomeric material will generally have a large Poisson lateral contraction effect, i.e., they will "neck down" as they elongate in response to an applied force. Web materials of the present invention can be designed to moderate if not substantially eliminate the Poisson lateral contraction effect.

For web material 52, the direction of applied axial elongation, D, indicated by arrows 80 in Fig. 3, is substantially perpendicular to the first axis 76 of the rib-like elements 74. This is due to the fact that the rib-like elements 74 are able to unbend or geometrically deform in a direction substantially perpendicular to their first axis 76 to allow extension in web 52.

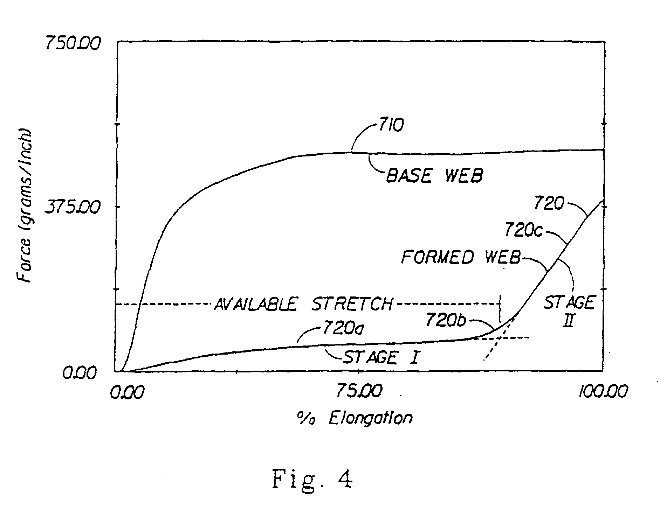

In Fig. 4 there is shown an exemplary graph of a resistive force-elongation curve 720 of a web material generally similar to web material 52 shown in Fig. 3 along with a curve 710 of a base web material of similar composition. The method for generating resistive force-elongation curves can be found in the Test Methods section set forth in subsequent portions of the present specification. Referring now to the force-elongation curve 720 of the formed web of the present invention, there is an initial substantially linear, lower force versus elongation stage I designated 720a, a transition zone designated 720b which indicates the encounter of the force wall, and a substantially linear stage II designated 720c which displays substantially higher force versus elongation behavior.

As seen in Fig. 4 the formed web exhibits different elongation behavior in the two stages when subjected to an applied elongation in a direction parallel to the transverse axis of the web. The resistive force exerted by the formed web to the applied elongation is significantly less in the stage I region (720a) versus the stage II region (720c) of curve 720. Furthermore, the resistive force exerted by the formed web to the applied elongation as depicted in stage 1 (720a) of curve 720 is significantly less than the resistive force exerted by the base web as depicted in curve 710 within the limits of elongation of stage I. As the formed web is subjected to further applied elongation and enters stage II (720c) the resistive force exerted by the formed web increases and approaches the resistive force exerted by the base web. The resistive force to the applied elongation for the stage I region (720a) of the formed web is provided by the molecular-level and geometric deformation of the first region of the formed web and the geometric deformation of the second region of the formed web. This is in contrast to the resistive force to an applied elongation that is provided by the base web, depicted in curve 710 of Fig. 4, which results from molecular-level deformation of the entire web. Web materials of the present invention can be designed to yield virtually any resistive force in stage I which is less than that of the base web material by adjusting the percentage of the web surface which is comprised of the first and second regions, respectively. The force-elongation behavior of stage I can be controlled by adjusting the width, cross-sectional area, and spacing of the first region and the composition of the base web.

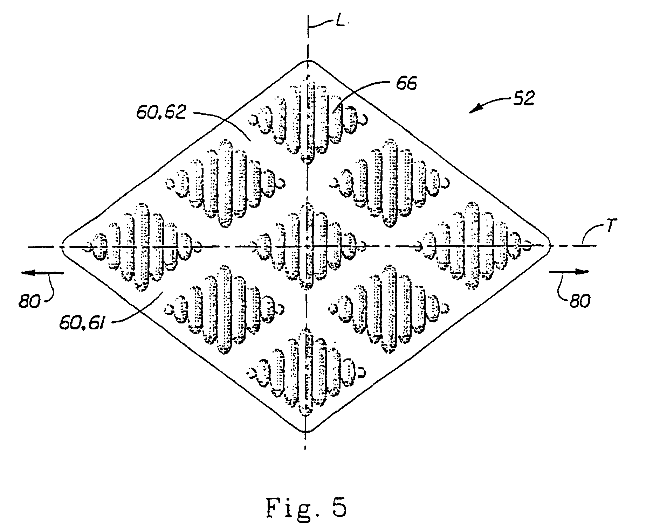

Referring now to Fig. 5, as web 52 is subjected to an applied axial elongation, D, indicated by arrows 80 in Fig. 5, the first regions 60 having the shorter surface-pathlength, L1, provide most of the initial resistive force, P1, as a result of molecular-level deformation, to the applied elongation which corresponds to stage I. While in stage I, the rib-like elements 74 in the second regions 66 are experiencing geometric deformation, or unbending and offer minimal resistance to the applied elongation. In addition, the shape of the second regions 66 changes as a result of the movement of the reticulated structure formed by the intersecting first regions 61 and 62. Accordingly, as the web 52 is subjected to the applied elongation, the first regions 61 and 62 experience geometric deformation or bending, thereby changing the shape of the second regions 66. The second regions are extended or lengthened in a direction parallel to the direction of applied elongation, and collapse or shrink in a direction perpendicular to the direction of applied elongation.

In the transition zone (720b) between stages I and II, the rib-like elements 74 are becoming aligned with, (i. e., coplanar with), the applied elongation. That is, the second region 66 is exhibiting a change from geometric deformation to molecular-level deformation. This is the onset of the force wall. In stage II, the rib-like elements 74 in the second region 66 are substantially aligned with, (i.e., coplanar with), the axis of applied elongation (i.e. the second region has reached its limit of geometric deformation) and begin to resist further elongation via molecular-level deformation. The second region 66 now contributes, as a result of molecular-level deformation, a second resistive force, P2, to further applied elongation. In stage II, the first regions 61 and 62 have also reached their limit of geometric deformation and resist further elongation mainly via molecular-level deformation. The resistive forces to elongation depicted in stage II by both the molecular-level deformation of the first regions 60 and the molecular-level deformation of the second regions 66 provide a total resistive force, PT, which is greater than the resistive force depicted in stage I which is provided by the molecular-level and geometric deformation of the first regions 60 and the geometric deformation of the second regions 66. Accordingly, the slope of the force-elongation curve in stage II is significantly greater than the slope of the force-elongation curve in stage I.

The resistive force P1 is substantially greater than the resistive force P2 when (L1 + D) is less than L2. When (L1 + D) is less than L2 the first region provides the initial resistive force P1, generally satisfying the equation:

$$P1 = \frac{(A1 \times E1 \times D)}{L1}$$

When (L1 + D) is greater than L2 the first and second regions provide a combined total resistive force PT to the applied elongation, D, generally satisfying the equation:

$$PT = \frac{(A1 \times E1 \times D)}{L1} + \frac{(A2 \times E2 \times [L1 + D - L2])}{L2}$$

The maximum elongation occurring while in stage I is referred to as the "available stretch" of the formed web material. The available stretch corresponds to the distance over which the second region experiences geometric deformation. The available stretch can be effectively determined by inspection of the force-elongation curve 720 as shown in Fig. 4. The approximate point at which there is an inflection in the transition zone between stage I and stage II is the percent elongation point of "available stretch". The range of available stretch can be varied from about 10% to 100% or more; this range of elongation is often found to be of interest in disposable absorbent articles, and can be largely controlled by the extent to which the surface-pathlength L2 in the second region exceeds the surface-pathlength L1 in the first region and the composition of the base film. The term available stretch is not intended to imply a limit to the elongation which the web of the present invention may be subjected to as there are applications where elongation beyond the available stretch is desirable.

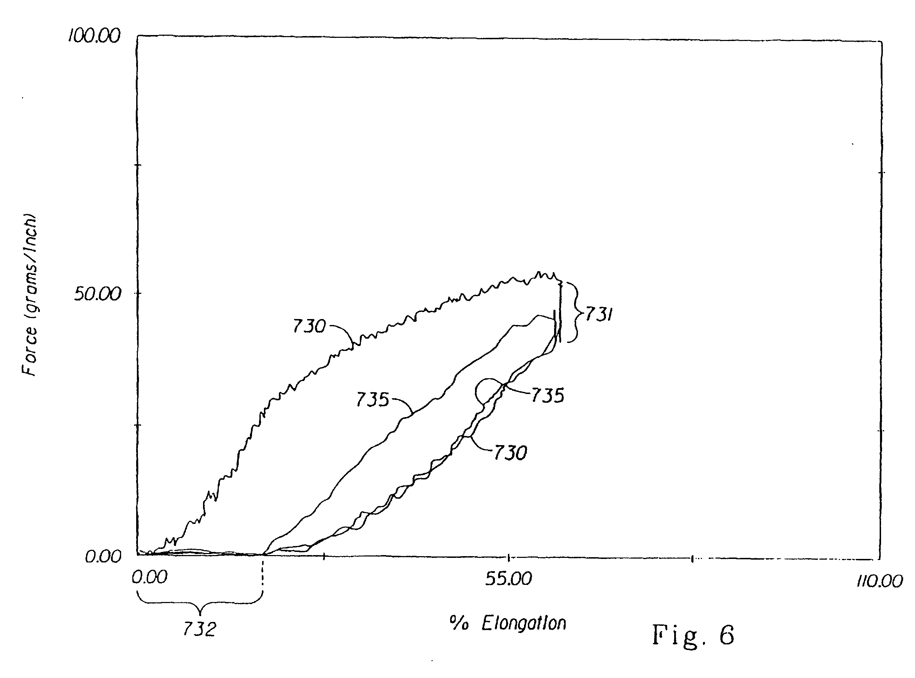

The curves 730 and 735 in Fig. 6 show an exemplary elastic hysteresis behavior exhibited by a web material of the present invention. Curve 730 represents the response to an applied and released elongation during the first cycle and curve 735 represent the response to an applied and released elongation during the second cycle. The force relaxation during the first cycle 731 and the percent set or deformation 732 are depicted in Fig. 6. Note that significant recoverable elongation, or useful elasticity, is exhibited at relatively low forces over multiple cycles, i.e., the web material can easily expand and contract to a considerable degree. The method for generating the elastic hysteresis behavior can be found in the Test Method section set forth in subsequent portion of the present specification.

When the web material is subjected to an applied elongation, the web material exhibits an elastic-like behavior as it extends in the direction of applied elongation and returns to its substantially untensioned condition once the applied elongation is removed, unless the web material is extended beyond the point of yielding. The web material is able to undergo multiple cycles of applied elongation without losing its ability to substantially recover. Accordingly, the web is able to return to its substantially untensioned condition once the applied elongation is removed.

While the web material may be easily and reversibly extended in the direction of applied axial elongation, in a direction substantially perpendicular to the first axis 76 of the rib-like elements 74, the web material is not as easily extended in a direction substantially parallel to the first axis 76 of the rib-like elements 74. The formation of the rib-like elements allows the rib-like elements to geometrically deform in a direction substantially perpendicular to the first or major axis 76 of the rib-like elements, while requiring substantially molecular-level deformation to extend in a direction substantially parallel to the first axis of the rib-like elements.

The amount of applied force required to extend the web is dependent upon the composition and cross-sectional area of the web material and the width and spacing of the first regions, with narrower and more widely spaced first regions requiring lower applied extensional forces to achieve the desired elongation for a given composition and cross-sectional area.

The depth and frequency of rib-like elements can also be varied to control the available stretch of a web of the present invention. The available stretch is increased if for a given frequency of rib-like elements, the height or degree of formation imparted on the rib-like elements is increased. Similarly, the available stretch is increased if for a given height or degree of formation, the frequency of the rib-like elements is increased.

There are several functional properties that can be controlled through the application of the present invention. The functional properties are the resistive force exerted by the web material against an applied elongation and the available stretch of the web material before a force wall is encountered. The resistive force that is exerted by the web material against an applied elongation is a function of the material (e.g., composition, molecular structure and orientation, etc.) and cross-sectional area and the percent of the projected surface area of the web material that is occupied by the first region. The higher the percent area coverage of the web material by the first region, the higher the resistive force that the web will exert against an applied elongation for a given material composition and cross-sectional area. The percent coverage of the web material by the first region is determined in part if not wholly by the widths of the first regions and the spacing between adjacent first regions.

The available stretch of the web material is determined by the surface-pathlength of the second region. The surface-pathlength of the second region is determined at least in part by the rib-like element spacing, rib-like element frequency and depth of formation of the rib-like elements as measured perpendicular to the plane of the web material. In general, the greater the surface-pathlength of the second region the greater the available stretch of the web material.

In addition to the aforementioned elastic-like properties, a plastic film of the present invention is also characterized as being soft, cloth-like in texture and appearance, and quiet. The soft, cloth-like, quiet, plastic film is also a liquid barrier making it especially useful as a backsheet on a disposable absorbent article, such as a disposable diaper. In general, the soft cloth-like texture creates a garment-like impression, which increases the aesthetic appeal of the disposable article.

While an entire web material of the present invention may include a strainable network of first and second regions, the present invention may also be practiced by providing only specific portions of the web with a strainable network comprised of first and second regions. It will be obvious to one skilled in the art that all or a portion of a backsheet on a disposable absorbent article may include a strainable network(s) comprised of first and second regions.

While the web material having a strainable network of the present invention has been described as a backsheet or a portion thereof on an absorbent article, in some embodiments it may be necessary to provide the topsheet and the absorbent core with a strainable network.

Method of Making

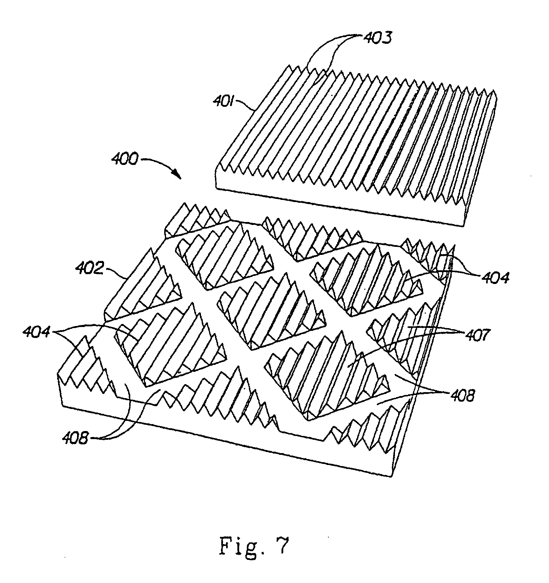

Referring now to Fig. 7, there is shown a portion of a texturing unit 400 used to form the web 52 shown in Fig. 3. The texturing unit 400 includes intermeshing rolls 401, 402. The rolls 401, 402 include a plurality of intermeshing teeth 403, 404, respectively. The rolls 401, 402 are brought together under pressure to form the web of the present invention.

Roll 402 includes toothed regions 407 and grooved regions 408. Within toothed regions 407 of roll 402 there are a plurality of teeth 404. Roll 401 includes teeth 403 which mesh with teeth 404 of roll 402. When a film is formed between rolls 401, 402 the portions of the film which are positioned within grooved regions 408 of roll 402 and teeth 403 on roll 401 remain undeformed. These regions correspond with the first regions 60 of web 52 shown in Fig. 3. The portions of the film positioned between toothed regions 407 of roll 402, (which comprise teeth 404), and teeth 403 of roll 401 are incrementally and plastically formed creating rib-like elements 74 in the second regions 66 of web material 52.

The method of formation can be accomplished as follows. A roll of untextured material is placed on a web tension control unwind stand. It is directed to the texturing unit by a series of supporting undriven idlers. The unwind unit will pay out material at sufficient velocity to supply the texturing apparatus and maintain the preselected web tension.

The texturing unit comprises two intermeshing rolls, a cross hatched texturing roll and a ring roll. They are both driven to have a matching surface speed at least 111 m/min(370 ft/min), in a direction that constitutes an in-running nip. The texturing rolls may be provided in various configurations. For example, pitch (distance between teeth), engagement depth, channel spacing, number of teeth, height of teeth, and areas of nonuniformity (for example, electropolishing to eliminate such areas) are parameters which may be varied.

After the material is textured, it is directed to a web tension control rewind stand, again by a series of supporting undriven rollers. The rewind unit will wind the material onto a roll at sufficient velocity to consume the material supplied by the texturing unit and maintain the preselected web tension.

The web tension is normally selected to correspond to web strain of about 0.75%. Tensions in the range of about 0.5% to about 1.5% are generally preferred.

If the axis of rotation of the rolls were placed such a distance apart that the lines of circumference intersected at one point, this would be denoted as zero engagement. If the two axes were moved closer together, the distance moved would be the new engagement.

Web materials of the present invention may be comprised of polyolefins such as polyethylenes, including linear low density polyethylene (LLDPE), low density polyethylene (LOPE), ultra low density polyethylene (ULDPE), high density polyethylene (HDPE), or polypropylene and blends thereof with the above and other materials, for example calcium carbonate, silica, or zeolite, in a variety of particle sizes. Examples of other suitable polymeric materials which may also be used include, but are not limited to, polyester, polyurethanes, compostable or biodegradable polymers, heat shrink polymers, thermoplastic elastomers, metallocene catalyst-based polymers (e.g., INSITE, available from Dow Chemical Company and Exxact, available from Exxon), and breathable polymers. The web material may also be comprised of synthetic woven, synthetic knit, nonwoven, apertured film, macroscopically expanded three-dimensional formed film, absorbent or fibrous absorbent material, foam, filled composition, a material of temporary wet strength, or laminates and/or combinations thereof. The nonwovens may be made by but not limited to any of the following methods: spunlace, spunbond, meltblown, carded and/or air-through or calendar bonded.

While the present invention has been described as providing a web material from a single layer of base film, the present invention may be practiced equally well with other materials. While the fluid impervious polymeric film exhibiting an elastic-like behavior in the direction of applied elongation may be suitable for use a backsheet on a disposable diaper or sanitary napkin, such a web material would not function well as a topsheet on an absorbent article. Examples of other base materials from which the web of the present invention can be made and will function effectively as a fluid pervious topsheet on an absorbent article include two-dimensional apertured films and macroscopically expanded, three-dimensional, apertured formed films. Examples of macroscopically expanded, three-dimensional, apertured formed films are described in U.S. Pat. No. 3,929,135, issued to Thompson on December 30, 1975; U.S. Pat. No. 4,324,246 issued to Mullane, et al. on April 13, 1982; U.S. Pat. No. 4,342,314 issued to Radel, et al. on August 3, 1982; U.S. Pat. No. 4,463,045 issued to Ahr, et al. on July 31, 1984; and U.S. Pat. No. 5.006,394 issued to Baird on April 9, 1991.

Web materials of the present invention may include laminates of the above mentioned materials. Laminates may be combined by any number of bonding methods known to those skilled in the art Such bonding methods include but are not limited to thermal bonding, adhesive bonding (using any of a number of adhesives including but not limited to spray adhesives, hot melt adhesives, latex based adhesives and the like), sonic bonding and extrusion laminating whereby a polymeric film is cast directly onto a substrate, and while still in a partially molten state, bonds to one side of the substrate, or by depositing meltblown fibers nonwoven directly onto a substrate.

Test Methods

MVTR

The MVTR is measured by the method set forth below. A known amount of calcium chloride ($CaCl_2$) is put

into a flanged cup. A sample is placed on the top of the cup and held securely by a retaining ring and gasket. The assembly is then weighed and recorded as the initial weight The assembly is placed in a constant temperature (40°C) and humidity (75% relative humidity) chamber for 5 hours. The assembly is then removed from the chamber an allowed to equilibrate for at least 30 minutes at the temperature of the room where the balance is located. The assembly is then weighed and recorded as the final weight. The MVTR is calculated and expressed in $g/m^2/24$ hr. using the following formula:

$$MVTR = \frac{(Final\ weight - initial\ weight \times 24.0}{Area\ of\ sample\ in\ meters \times 5.0\ (time\ in\ chamber)}$$

The web materials of the present invention preferably have an MVTR of at least about 100 grams/$m^2$/24 hrs., with at least about 1500 grams/$m^2$/24 hrs. being more preferable for diapers. Preferably, the MVTR is as high as possible with no leaks.

MWTR

MWTR may be measured by the following method, designed to quantify the maximum amount of water transported through microholes in a sample web material, for example the backsheet of a disposable diaper. The initial weight of the diaper to be tested is recorded. A cradle-type boat having dimensions depending on the size of the diaper to be tested is covered by the core area of the diaper. A basin of dimensions 400 mm x 500 mm is filled with colored water, and the cradle with the diaper is placed in the basin. A 2,25 kg (5 pound) weight is placed on the diaper. The height of the water level in the basin should not exceed 13mm (0.5 in.). After 20 minutes, the diaper is weighed again. The MWTR is calculated and expressed in grams of water/hr. using the following formula:

$$MWTR = (Wet\ weight - dry\ weight) \times 3.0$$

For disposable article incorporating the web materials of the present invention, preferably the MWTR for a single article is no greater than about 100 grams/hr; or expressed for a sample size of 10 articles, no greater than about 50 g/hr.

Surface-Pathlength

Pathlength measurements of formed material regions are to be determined by selecting and preparing representative samples of each distinct region and analyzing these samples by means of microscopic image analysis methods.

Samples are to be selected so as to be representative of each region's surface geometry. Generally, the transition regions should be avoided since they would normally contain features of both the first and second regions. The sample to be measured is cut and separated from the region of interest. The "measured edge" is to be cut parallel to a specified axis of elongation. An unstrained sample length of one-half inch is to be "gauge marked" perpendicular to the "measured edge": while attached to the web material, and then accurately cut and removed from the web material.

Measurement samples are then mounted onto the long-edge of a microscopic glass slide. The "measured edge" is to extend slightly (approximately 1 mm) outward from the slide edge. A thin layer of pressure-sensitive adhesive is applied to the glass face-edge to provide a suitable sample support means. For highly formed sample regions it has been found desirable to gently extend the sample in its axial direction (without imposing significant force) simultaneous to facilitate contact and attachment of the sample to the slide-edge. This allows improved edge identification during image analysis and avoids possible "crumpled" edge portions that require additional interpretation analysis.

Images of each sample are to be obtained as "measured edge" views taken with the support slide "edge on" using suitable microscopic measuring means of sufficient quality and magnification. Data is obtained using the following equipment; Keyence VH-6100 (20x Lens) video unit, with video-image prints made with a Sony Video printer Mavigraph unit. Video prints were image-scanned with a Hewlett Packard ScanJet IIP scanner. Image analysis was on a Macintosh IICi computer utilizing the software NIH MAC Image version 1.45.

Using this equipment, a calibration image initially taken of a grid scale length of .500" with .005" increment-marks to be used for calibration setting of the computer image analysis program. All samples to be measured are then video-imaged and video-image printed. Next, all video-prints are image-scanned at 100 dpi (256-level gray scale) into a suitable Mac image-file format. Finally, each image-file (including calibration file) is analyzed utilizing Mac Image 1.45 computer program. All samples are measured with freehand line-measurement tool selected. Samples are measured on both side-edges and the lengths are recorded. Simple film-like (thin & constant thickness) samples require only one side-edge to be measured. Laminate and thick foam samples are measured on both side-edges. Length measurement tracings are to be made along the full gauge length of a cut sample. In cases of highly deformed samples, multiple (partially overlapping) images may be required to cover the entire cut sample. In these cases, select characteristic features common to both overlapping-images and utilize as "markers" to permit image length readings to adjoin but not overlap.

The final determination of surface-pathlength for each region is obtained by averaging the lengths of five (5) separate 1/2" gauge-samples of each region. Each gauge-sample "surface-pathlength" is to be the average of both side-edge surface-pathlengths.

Poisson's Lateral Contraction Effect

The Poisson's lateral contraction effect is measured cn an Instron Model 1122, as available from Instron Corporation of Canton, Massachusetts, which is interfaced to a Gateway 2000 486/33Hz computer available from Gateway 2000 of N. Sioux City, South Dakota, using Test Works‰ software which is available from Sintech, Inc. of Research Triangle Park, North Carolina. All essential parameters needed for testing are input in the TestWorks‰ software for each test. Data collection is accomplished through a combination of manual sample width measurements, and elongation measurements made within TestWorks‰.

The samples used for this test are 1" wide x 4" long with the long axis of the sample cut parallel to the direction of the first region of the sample. The sample should be cut with a sharp knife or suitably sharp cutting device designed to cut a precise 1" wide sample. It is important that a "representative sample" should be cut so that an area representative of the symmetry of the overall pattern of the deformed region is represented. There will be cases (due to variations in either the size of the deformed portion or the relative geometries of regions 1 and 2) in which it will be necessary to cut either larger or smaller samples than is suggested herein. In this case, it is very important to note (along with any data reported) the size of the sample, which area of the deformed region it was taken from and preferably include a schematic of the representative area used for the sample. In general, an "aspect ratio" of (2:1) for the actual extended tensile portion (l1:w1) is to be maintained if possible. Five samples are tested.

The grips of the Instron consist of air actuated grips designed to concentrate the entire gripping force along a single line perpendicular to the direction of testing elongation having one flat surface and an opposing face from which protrudes a half round. No slippage should be permitted between the sample and the grips. The distance between the lines of gripping force should be 2" as measured by a steel rule held beside the grips. This distance will be referred to from here on as the "gauge length".

The sample is mounted in the grips with its long axis perpendicular to the direction of applied elongation. An area representative of the overall pattern geometry should be symmetrically centered between the grips. The crosshead speed is set to 10 in/min. The crosshead moves to the specified strain (measurements are made at both 20 and 60% elongation). The width of the sample at its narrowest point (w2) is measured to the nearest 0.02" using a steel rule. The elongation in the direction of applied extension is recorded to the nearest 0.02" on the TestWorks software. The Poisson's Lateral Contraction Effect (PLCE) is calculated using the following formula:

$$PLCE = \frac{\frac{|w2 - W1|}{w1}}{\frac{|l2 - l1|}{l1}}$$

where

    w2 = The width of the sample under an applied longitudinal elongation;
    w1 = The original width of the sample;
    l2 = The length of the sample under an applied longitudinal elongation; and
    11 = The original length of the sample (gauge length);

Measurements are made at both 20 and 60% elongation using five different samples for each given elongation. The PLCE at a given percent elongation is the average of five measurements.

Hysteresis Test

The hysteresis test is used for measuring the percent set and percent force relaxation of a material. The tests are performed on an Instron Model 1122, available from Instron Corporation of Canton, Mass. which is interfaced to a Gateway 2000 486/33Hz computer available from Gateway 2000 of N. Sioux City, South Dakota 57049, using Test-

Works‰ software which is available from Sintech, Inc. of Research Triangle Park, North Carolina 27709. All essential parameters needed for testing are input in the TestWorks‰ software for each test (i.e. Crosshead Speed, Maximum percent elongation Point and Hold Times). Also, all data collection, data analysis and graphing are done using the TestWorks‰ software.

The samples used for this test are 1" wide x 4" long with the long axis of the sample cut parallel to the direction of maximum extensibility of the sample. The sample should be cut with a sharp exacto knife or some suitably sharp cutting device design to cut a precise 1" wide sample. (If there is more than one direction of elongation of the material, samples should be taken parallel to representative directions of elongation). The sample should be cut so that an area representative of the symmetry of the overall pattern of the deformed region is represented. There will be cases (due to variations in either the size of the deformed portion or the relative geometries of the first and second regions) in which it will be necessary to cut either larger or smaller samples than is suggested herein. In this case, it is very important to note (along with any data reported) the size of the sample, which area of the deformed region it was taken from and preferably include a schematic of the representative area used for the sample. Three separate tests at 20, 60 and 100% strain are typically measured for each material. Three samples of a given material are tested at each percent elongation.

The grips of the Instron consist of air actuated grips designed to concentrate the entire gripping force along a single line perpendicular to the direction of testing stress having one flat surface and an opposing face from which protrudes a half round to minimize slippage of the sample. The distance between the lines of gripping force should be 2" as measured by a steel rule held beside the grips. This distance will be referred to from hereon as the "gauge length". The sample is mounted in the grips with its long axis perpendicular to the direction of applied percent elongation. The crosshead speed is set to 10 in/min. The crosshead moves to the specified maximum percent elongation and holds the sample at this percent elongation for 30 seconds. After the thirty seconds the crosshead returns to its original position (0% elongation) and remains in this position for 60 seconds. The crosshead then returns to the same maximum percent elongation as was used in the first cycle, holds for thirty seconds and then again returns to zero.

A graph of two cycles is generated. The percent force relaxation is determined by the following calculation of the force data from the first cycle:

$$\frac{\text{Force at Max. \% elongation - Force after 30 sec. hold x 100}}{\text{Force at Maximum \% elongation (cycle 1)}} = \text{\% Force Relaxation}$$

The percent set is the percent elongation of the sample of the second cycle where the sample starts to resist the elongation. The average percent force relaxation and percent set for three samples is reported for each maximum percent elongation value tested.

Tensile Test

The tensile test is used for measuring force versus percent elongation properties and percent available stretch of a material. The tests are performed on an Instron Model 1122, available from Instron Corporation of Canton, Mass. which is interfaced to a Gateway 2000 486/33Hz computer available from Gateway 2000 of N. Sioux City, South Dakota , using TestWorks‰ software which is available from Sintech, Inc. of Research Triangle Park, North Carolina. All essential parameters needed for testing are input in the TestWorks‰ software for each test. Also, all data collection, data analysis and graphing are done using the TestWorks™ software.

The samples used for this test are 1" wide x 4" long with the long axis of the sample cut parallel to the direction of maximum extensibility of the sample. The sample should be cut with a sharp exacto knife or some suitably sharp cutting device design to cut a precise 1" wide sample. (If there is more than one direction of extensibility of the material, samples should be taken parallel to representative direction of elongation). The sample should be cut so that an area representative of the symmetry of the overall pattern of the deformed region is represented. There will be cases (due to variations in either the size of the deformed portion or the relative geometries of regions 1 and 2) in which it will be necessary to cut either larger or smaller samples than is suggested herein. In this case, it is very important to note (along with any data reported) the size of the sample, which area of the deformed region it was taken from and preferably include a schematic of the representative area used for the sample. Three samples of a given material are tested.

The grips of the Instron consist of air actuated grips designed to concentrate the entire gripping force along a single line perpendicular to the direction of testing stress having one flat surface and an opposing face from which protrudes a half round to minimize slippage of the sample. The distance between the lines of gripping force should be 2" as measured by a steel rule held beside the grips. This distance will be referred to from hereon as the "gauge length". The sample is mounted in the grips with its long axis perpendicular to the direction of applied percent elongation. The crosshead speed is set to 10 in/min. The crosshead elongates the sample until the sample breaks at which point the crosshead stops and returns to its original position (0 % elongation).

The percent available stretch is the point at which there is an inflection in the force - elongation curve, beyond which point there is a rapid increase in the amount of force required to elongate the sample further. The average of the percent available stretch for three samples is recorded.

**EXAMPLES**

The following examples further describe and demonstrate the preferred embodiments within the scope of the present invention. The examples are given solely for the purposes of illustration, and are not to be construed as limitations of the present invention since many variations thereof are possible without departing from its spirit and scope.

The materials of the present invention are suitably made as set forth in the Method of Making section of this description. The following examples illustrate line speed of about 225 m/min (750 ft/min).

Table 1.1

| 30 Pitch Texturing Roll *1 | | | | | | | |
|---|---|---|---|---|---|---|---|
| Material Description | TXB (in) *2 | Average MWTR (g H2O/hr) | n = | Average MVTR (g H2O/ day/ /m2) | n = | Average MWTR (g H2O/day/ m2) | MVTR/ MWTR |
| Tredegar 1.2 mil film/NW *3 | 0.025" | 7.16 | 20 | 2880 | 7 | 5633 | 0.511 |
| " | 0.030" | 28.591 | 10 | 3656 | 5 | 22497 | 0.163 |
| 1.2 mil MD Weave Precursor (Tredegar) *4 | 0.025" | 13.73 | 10 | 2822 | 5 | 10801 | 0.261 |
| 1.1 mil film/ NW (Tredegar) *5 | 0.025" | 36.54 | 10 | 2669 | 3 | 28750 | 0.093 |
| " | 0.030" | 89.24 | 10 | 2872 | 3 | 70225 | 0.041 |
| " | 0.035" | 75.33 | 10 | 3092 | 3 | 59277 | 0.052 |
| 1.2 mil film/ NW (Clopay) *6 | 0.025" | 7.73 | 10 | 1381 | 3 | 6082 | 0.227 |
| " | 0.030" | 23.60 | 10 | 1987 | 3 | 18570 | 0.107 |
| 1.2 mil Precursor (Clopay) *7 | 0.025" | 7.85 | 10 | 2046 | 3 | 6176 | 0.331 |
| " | 0.030" | 12.36 | 10 | 2411 | 3 | 9729 | 0.248 |
| " | 0.035" | 86.72 | 10 | 2773 | 3 | 68241 | 0.041 |

Table 1.2

| 30 Pitch Electro-polished Texturing Roll | | | | | | | |
|---|---|---|---|---|---|---|---|
| Material Description | TXB (in) *2 | Average MWTR (g H2O/hr) | n = | Average MVTR (g H2O/ day/ /m2) | n = | Average MWTR (g H2O/day/ m2) | MVTR/ MWTR |
| Tredegar 1.2 mil film/NW *3 | 0.030" | 12.10 | 10 | 3345 | 3 | 9522 | 0.351 |
| 1.1 mil film/ NW (Tredegar) *5 | 0.025" | 1.87 | 10 | 2365 | 3 | 1473 | 1.606 |
| " | 0.035" | 9.45 | 10 | 3361 | 3 | 7433 | 0.452 |

Table 1.2   (continued)

| 30 Pitch Electro-polished Texturing Roll | | | | | | | |
|---|---|---|---|---|---|---|---|
| Material Description | TXB (in) *2 | Average MWTR (g H2O/hr) | n = | Average MVTR (g H2O/ day/ /m2) | n = | Average MWTR (g H2O/day/ m2) | MVTR/ MWTR |
| 1.2 mil film/ NW (Clopay) *6 | 0.025" | 3.66 | 10 | 1575 | 3 | 2880 | 0.547 |

Table 1.3

| 40 Pitch Texturing Roll | | | | | | | |
|---|---|---|---|---|---|---|---|
| Material Description | TXB (in) *2 | Average MWTR (g H2O/hr) | n = | Average MVTR (g H2O/ day/ /m2) | n = | Average MWTR (g H2O/ day/ m2) | MVTR/ MWTR |
| Tredegar 1.2 mil film/NW *3 | 0.040" | 14.07 | 10 | 3037 | 3 | 11071 | 0.274 |
| 1,1 mil film/ NW (Tredegar) *5 | 0.030" | 2.89 | 10 | 2245 | 3 | 2273 | 0.988 |
| " | 0.050" | 13.30 | 10 | 3243 | 3 | 10466 | 0.310 |
| 1.2 mil film/ NW (Clopay) *6 | 0.030" | 5.89 | 10 | 1150 | 3 | 4632 | 0.248 |

*1 Pitch of teeth on roll *2 Amount of engagement of teeth rolls

*3 Supplier ID No. X-15918, Tredegar Film Products, Richmond, VA USA ("Film/nw" refers to film nonwoven laminate) *4 Supplier ID No. X-11025

*5 Supplier ID No. X-15910

*6 Supplier ID No. P18-3587 x 0.0012, Clopay Plastic Products, Cincinnati, OH USA *7 Supplier ID No. P18-3551 x 0.0012

The embodiments disclosed and represented by the previous examples have many advantages. For example, they can provide breathability and soft cloth-like texture without substantial leakage. Further, they are cost effective to manufacture as they are rendered breathable by full speed on-line activation without the need to hot tenter as separate process.

**Claims**

1.   A soft web material (52) comprising: a plurality of first regions (60) and a plurality of second regions (66) being comprised of the same material composition, the first regions (60) surrounding the second regions (66) and the second regions comprising a plurality of raised rib-like elements (74) **characterized in that**
the web material comprises, microporous holes in the second regions (66) and the web material (52) exhibits a MVTR of at least about 100 $g/m^2/24$ hrs and a MWTR of not greater than about 100 g/hr.

2.   The web material of Claim 1, wherein said web material (52) is a backsheet (36) on a disposable absorbent article (20,30).

3.   The web material of Claim 4, wherein said web material (52) is a laminate of two or more materials.

4.   The web material of Claim 1, wherein the web material (52) has zones of variable MVTR.

**Patentansprüche**

1. Weiches Gewebe-Material (52) umfassend: eine Vielzahl erster Bereiche (60) und eine Vielzahl zweiter Bereiche (66), die aus der gleichen Material-Zusammensetzung bestehen, wobei die ersten Bereiche (60) die zweiten Bereiche (66) umgeben und die zweiten Bereiche eine Vielzahl erhöhter, rippenartiger Elemente (74) aufweisen, **dadurch gekennzeichnet, dass** das Gewebe-Material mikroporöse Löcher in den zweiten Bereichen (66) umfasst, und das Gewebe-Material (52) ein MVTR von mindestens ungefähr 100 g/m$^2$/24h und ein MWTR von nicht mehr als ungefähr 100 g/h aufzeigt.

2. Gewebe-Material nach Anspruch 1, wobei das Gewebe-Material (52) eine Außenlage (36) auf einem wegwerfbaren, absorbierenden Artikel (20, 30) ist.

3. Gewebe-Material nach Anspruch 2, wobei das Gewebe-Material (52) ein Schichtstoff aus zwei oder mehreren Materialien ist.

4. Gewebe-Material nach Anspruch 1, wobei das Gewebe-Material (52) Zonen mit unterschiedlichem MVTR aufweist.


**Revendications**

1. Nappe douce (52) comprenant une pluralité de premières régions (60) et une pluralité de deuxièmes régions (66) ayant la même composition de matériau, les premières régions (60) entourant les deuxièmes régions (66) et les deuxièmes régions comprenant une pluralité d'éléments (74) en saillie du type nervure, **caractérisée en ce que** la nappe comprend des trous microporeux dans les deuxièmes régions (66), et la nappe (52) présente une MVTR d'au moins environ 100 g/m$^2$ et une MWTR non supérieure à environ 100 g/h.

2. Nappe selon la revendication 1, dans laquelle ladite nappe (52) est une feuille de fond (36) sur un article absorbant jetable (20, 30).

3. Nappe selon la revendication 2, dans laquelle ladite nappe (52) est un stratifié de deux matériaux ou plus.

4. Nappe selon la revendication 1, dans laquelle la nappe (52) a des zones de MVTR variables.

FIG. 1

( PRIOR ART )

Fig. 2

( PRIOR ART )

Fig. 3

Fig. 4

Fig. 5

EP 1 040 217 B1

Fig. 6

Fig. 7